# EUROPÄISCHE PATENTSCHRIFT

(11) **EP 2 560 114 B1**
(45) Veröffentlichungstag und Bekanntmachung des Hinweises auf die Patenterteilung: **05.07.2017**
(21) Anmeldenummer: 11177712.4
(22) Anmeldetag: 16.08.2011
(51) Int. Cl.: G06F 19/00

(54) **Vorrichtung zum Unterstützen eines Patienten mit einer chronischen oder einer nicht-chronischen Krankheit und Verfahren zum Betreiben der Vorrichtung**
Device for supporting a patient with a chronic or non-chronic illness and method for operating the device
Dispositif d'assistance d'un patient ayant une maladie chronique ou non chronique et procédé de fonctionnement du dispositif

(43) Veröffentlichungstag der Anmeldung: 20.02.2013
(73) Patentinhaber: Roche Diabetes Care GmbH, 68305 Mannheim (DE); F. Hoffmann-La Roche AG, 4070 Basel (CH)
(72) Erfinder: Essenpreis, Matthias, Dr., 69469 Weinheim (DE); Voelkel, Dirk, Dr., 69469 Weinheim (DE); Weinert, Stefan, Pendleton, IN 46064 (US)
(74) Vertreter: Bittner, Thomas L.

(56) Entgegenhaltungen:
- EP-A1- 2 348 470
- WO-A2-2010/072386
- US-A1- 2002 029 161

## Beschreibung

Die Erfindung betrifft eine Vorrichtung zum Unterstützen eines Patienten mit einer chronischen oder einer nicht-chronischen Krankheit, insbesondere Diabetes, und ein Verfahren zum Betreiben der Vorrichtung.

### Hintergrund der Erfindung

Derartige Vorrichtungen werden bereitgestellt, um dem Patienten den Umgang mit und die Bewältigung der Krankheit zu erleichtern. Darüber hinaus dienen derartige Vorrichtungen aber auch dem behandelndem Arzt dazu, diagnostische oder therapeutische Maßnahmen unterstützend zu begleiten. Hierbei greifen die Vorrichtungen nicht in die Diagnostik oder Therapie selbst ein oder sind hieran unmittelbar beteiligt, sondern dienen insbesondere dazu, elektronische Informationen, insbesondere in Form von Messdaten, zu sammeln, zu verarbeiten und hieraus ableitend Datenausgaben zu bewirken.

Aus dem Dokument WO 2010 / 072386 A2 sind ein strukturiertes Messverfahren für diagnostische oder therapeutische Zwecke für einen Patienten mit einer chronischen Krankheit sowie eine Vorrichtung zur Nutzung des Verfahrens bekannt. Es wird vorgeschlagen, mittels einer auf einer Vorrichtung implementierten Applikation automatisch ein strukturiertes Sammelprotokoll zur diagnostischen oder therapeutischen Unterstützung eines Patienten auszuwählen und dieses zu implementieren, wobei das strukturierte Sammelprotokoll anhand von zugeordneten Parametern die Sammelprozedur definiert. Zu den Parametern gehört beispielsweise ein Zeitplan für Ereignisse zur Messdatenerfassung.

Das Dokument US 2008 / 0201174 A1 offenbart ein personalisiertes medizinisches Managementsystem, mit dem automatisch Erinnerungen für einen Benutzer gesetzt und erzeugt werden können, die durch ein Ereignis ausgelöst sind. So kann im Lichte eines anstehenden Arztbesuches eine automatische Erinnerung an den Benutzer ergehen, die auf spezifische Erfordernisse in Verbindung mit dem zukünftigen Termin hinweist. So kann der Benutzer daran erinnert werden, dass er zu dem Arztbesuch nüchtern erscheinen soll.

Das Dokument US 2003 / 0211617 A1 offenbart ein Verfahren sowie eine Vorrichtung zum Implementieren eines Blutzuckermessgerätes, welches den Benutzer an die notwendige Messung des Blutzuckerwertes erinnert. Dieses erfolgt in Relation zu einem zuvor erfassten Messwert.

### Zusammenfassung der Erfindung

Aufgabe der Erfindung ist es, verbesserte Technologien zum Unterstützen eines Patienten mit einer chronischen oder einer nicht-chronischen Krankheit, insbesondere Diabetes, anzugeben, mit der diagnostische oder therapeutische Maßnahmen in verbesserter Art und Weise unterstützend begleitet werden können.

Diese Aufgabe wird erfindungsgemäß durch eine Vorrichtung zum Unterstützen eines Patienten mit einer chronischen oder einer nicht-chronischen Krankheit nach dem unabhängigen Anspruch 1 sowie ein Verfahren zum Betreiben der Vorrichtung nach dem unabhängigen Anspruch 15 gelöst. Vorteilhafte Ausgestaltungen der Erfindung sind Gegenstand von abhängigen Unteransprüchen.

Die Erfindung umfasst den Gedanken einer Vorrichtung zum Unterstützen eines Patienten mit einer chronischen oder einer nicht-chronischen Krankheit, insbesondere Diabetes, mit einer Anzeigeeinrichtung, einer Benutzerschnittstelle, einem Prozessor, der an die Anzeigeeinrichtung und die Benutzerschnittstelle datentechnisch koppelt, und einem Applikationsmodul mit Programmanweisungen, die konfiguriert sind, beim Abarbeiten durch den Prozessor Folgendes zu bewirken:
- Bereitstellen von medizinischen Protokollen für diagnostische und / oder therapeutische Zwecke,
- Erfassen einer Zeitinformation betreffend ein zukünftiges Ereignis, welches Auslöser für zumindest ein medizinisches Protokoll ist,
- Auswählen eines der medizinischen Protokolle, welches eine Gruppe von zusammenhängenden Protokollereignissen angibt, die über einen Protokollzeitraum einem Protokollregime entsprechend zeitlich beabstandet sind,
- Überwachen eines zeitlichen Abstandes zwischen einem aktuellen Datum und dem zukünftigen Ereignis und
- Ausgeben eines Benutzer-Starthinweises betreffend das Beginnen des medizinischen Protokolls zu einem Zeitpunkt, welcher in zeitlicher Hinsicht wenigstens um den Protokollzeitraum vor dem zukünftigen Ereignis liegt,
- Berücksichtigung benutzerspezifischer Informationen, die zuvor vom Patienten angefordert wurden oder bereits in elektronischer Form vorliegen, wobei hierbei zum Protokollzeitraum eine Zeitdauer addiert wird. Nach einem weiteren Aspekt der Erfindung ist ein Verfahren zum Betreiben einer Vorrichtung zur Unterstützung eines Patienten mit einer chronischen oder einer nicht-chronischen Krankheit, insbesondere Diabetes, mit einer Anzeigeeinrichtung, einer Benutzerschnittstelle, einem Prozessor, der an die Anzeigeeinrichtung und die Benutzerschnittstelle datentechnisch koppelt, und einem Applikationsmodul mit Programmanweisungen geschaffen, die konfiguriert sind, wobei das Verfahren beim Abarbeiten der Programmanweisungen durch den Prozessor die folgenden Schritte umfasst:
   - Bereitstellen von medizinischen Protokollen für diagnostische und / oder therapeutische Zwecke,
   - Erfassen einer Zeitinformation betreffend ein zukünftiges Ereignis, welches Auslöser für zumindest ein medizinisches Protokoll ist,
   - Auswählen eines der medizinischen Protokolle, welches eine Gruppe von zusammenhängenden Protokollereignissen angibt, die über einen Protokollzeitraum einem Protokollregime entsprechend zeitlich beabstandet sind,
   - Überwachen eines zeitlichen Abstandes zwischen einem aktuellen Datum und dem zukünftigen Ereignis und
   - Ausgeben eines Benutzer-Starthinweises betreffend das Beginnen des medizinischen Protokolls zu einem Zeitpunkt, welcher in zeitlicher Hinsicht wenigstens um den Protokollzeitraum vor dem zukünftigen Ereignis liegt,
   - Berücksichtigung benutzerspezifischer Informationen, die zuvor vom Patienten angefordert wurden oder bereits in elektronischer Form vorliegen, wobei hierbei zum Protokollzeitraum eine Zeitdauer addiert wird.

In Abhängigkeit von einem zukünftigen Ereignis, zum Beispiel einem medizinischen Konsultationstermin wie einem Arzttermin, für das in elektronischer Form Zeitdaten gespeichert sind, die das zukünftige Ereignis in terminlicher oder zeitlicher Hinsicht charakterisieren, und von einem ausgewählten medizinischen Protokoll wird der Benutzer der Vorrichtung, also ein Patient mit einer chronischen oder nicht-chronischen Krankheit, automatisch an den Beginn einer vor dem zukünftigen Ereignis, also zum Beispiel dem Arzttermin, notwendigen Protokolldurchführung oder -abarbeitung nach dem Protokollregime hingewiesen. Das medizinische Protokoll ist Auslöser oder Anlass für das Durchführen des medizinischen Protokolls.

Bei dem medizinischen Protokoll kann es sich beispielsweise um ein strukturiertes Messwerterfassungsprotokoll handeln, welches eine Gruppe von zusammenhängenden Messereignissen angibt, die über einen Messzeitraum einem Messregime entsprechend zeitlich beabstandet sind. Aber auch die alternative oder ergänzende Protokollierung anderer Ereignisse kann Gegenstand des medizinischen Protokolls sein, wozu beispielsweise die Protokollierung von sportlichen Aktivitäten und / oder die Nahrungsaufnahme durch den Patienten gehören können.

Der Termin für das zukünftige Ereignis einerseits und das medizinische Protokoll andererseits bestimmen den Zeitpunkt für das Erzeugen und das Ausgeben des Benutzer-Starthinweises, mit dem der Benutzer auf den Beginn des medizinischen Protokolls hingewiesen wird. Wesentlich ist hierbei, dass das Ausgeben des Benutzer-Starthinweises rechtzeitig zu einem Zeitpunkt erfolgt, welcher in zeitlicher Hinsicht zumindest um den Protokollzeitraum vor dem Termin für das zukünftige Ereignis liegt, so dass der Zeitpunkt für die Ausgabe des Benutzer-Starthinweises vom ausgewählten medizinischen Protokoll abhängt.

Auf diese Weise wird zum Beispiel im Fall eines Messwerterfassungsprotokolls sichergestellt, dass der Benutzer die für das zukünftige Ereignis, bei dem es sich zum Beispiel um einen medizinischen Konsultations- oder Arzttermin handelt und welches für das Erfassungsprotokoll auslösend ist, notwendigen Messdaten rechtzeitig und in der gewünschten strukturierten Form misst und erfasst. Die Messdaten selbst kann der Patient auf beliebige Art und Weise sammeln, beispielsweise auch dadurch, dass er diese in ein Papierprotokoll dem strukturierten Messwerterfassungsprotokoll entsprechend notiert. Aber auch das Speichern der Messwerte in elektronischer Form in einem beliebigen Speichermedium kann vorgesehen sein.

Das automatische Ausgeben des Benutzer-Starthinweises unterstützt zum Beispiel diagnostische oder therapeutische Maßnahmen des Arztes oder anderen medizinischen Personals, indem beispielsweise vor einem anstehenden Arztbesuch der Patient angeleitet und geführt wird, ein gewünschtes Protokoll auszuführen, also zum Beispiel die gewünschten Messwerte zu erfassen, und zwar genau in der Weise wie es das strukturierte Messwerterfassungsprotokoll vorgibt.

Sofern es sich bei dem medizinischen Protokoll um ein Messwerterfassungs- oder Messwertprotokoll handelt, kann es sich bei den erfassten Messwerten insbesondere um Blutzuckermesswerte eines Diabetespatienten handeln. Insbesondere bei solchen Patienten ist es von großer Bedeutung, die Messwerte strukturiert, d. h. einem vorgegebenem Regime entsprechend, und in zeitlicher Hinsicht koordiniert zu erfassen. Nur so kann zum Beispiel der behandelnde Arzt Rückschlüsse aus den erfassten Messdaten ableiten, um gegebenenfalls geänderte oder neue therapeutische Maßnahmen vorzuschlagen.

Das Ausgeben des Benutzer-Starthinweises erfolgt beispielsweise über die Anzeigeeinrichtung der Vorrichtung. So kann ein visueller Hinweis mit entsprechendem Text über die Anzeigeeinrichtung ausgegeben werden. Ergänzend oder alternativ kann die Ausgabe des Benutzer-Starthinweises mittels einer akustischen und / oder einer taktilen Signalisierung vorgesehen sein. Letzteres kann zum Beispiel einen Vibrationsalarm umfassen.

Es kann aber auch vorgesehen sein, den Benutzer-Starthinweis über eine Schnittstelle an ein anderes Ausgabegerät zu übertragen, sei es über eine kabelgebundene oder eine drahtlose Datenverbindung. Auf diese Weise ist es ermöglicht, das Applikationsmodul zum Beispiel in einer Servereinrichtung zu implementieren, die über eine entsprechende Datenschnittstelle dann den Benutzer-Starthinweis an ein Benutzergerät überträgt, zum Beispiel einen Laptop, ein Mobilfunkgerät oder ein Blutzuckermessgerät. Insoweit können Prozessor und Anzeigeeinrichtung auch in getrennten Geräten implementiert sein, die datentechnisch gekoppelt sind, weshalb in dieser und anderen Ausgestaltungen auch die Bezeichnung System zum Unterstützen eines Patienten mit einer chronischen oder einer nicht-chronischen Krankheit, insbesondere Diabetes, zutreffend ist.

Die Auswahl des medizinischen Protokolls, beispielsweise des strukturierten Messwerterfassungsprotokolls, erfolgt benutzerspezifisch, wobei in diesem Zusammenhang vorgesehen sein kann, über die Benutzerschnittstelle erfasste Zusatzinformationen auszuwerten, die zum Beispiel vom behandelnden Arzt vorgegeben werden. Es kann aber auch vorgesehen sein, im Rahmen des Applikationsmoduls vor der Auswahl des medizinischen Protokolls vom Patienten Benutzerdaten zu erfassen, zum Beispiel Alter und / oder Gewicht, um hiervon ausgehend ein zugeordnetes strukturiertes Protokoll patientenspezifisch auszuwählen.

In einer Ausgestaltung kann vorgesehen sein, dem Benutzer die Möglichkeit zu geben, ein ausgewähltes medizinisches Protokoll nach der Auswahl aus einer Gruppe von Protokollen, zum Beispiel einer Menge von medizinischen Standardprotokollen, anzupassen oder zu ändern. So kann der Benutzer zum Beispiel selbst zusätzliche Protokollereignisse hinzufügen. Auf diese Weise ist eine weitergehende benutzerspezifische Ausbildung des Protokollregimes ermöglicht.

Eine Menge medizinischer Protokolle kann in einem Speicher elektronisch gespeichert sein, der mit dem Prozessor in derselben Geräteeinheit implementiert ist. Aber auch ein Fernzugriff des Prozessors auf eine Servereinrichtung mit mehreren medizinischen Protokollen über eine drahtlose oder eine kabelgebundene Datenkommunikationsverbindung kann in einer Ausgestaltung vorgesehen sein.

Der Zeitpunkt für das Ausgeben des Benutzer-Starthinweises kann bereits im medizinischen Protokoll selbst vorgegeben sein.

Es kann vorgesehen sein, die dem medizinischen Protokoll entsprechend erfassten Informationen, insbesondere Messwerte, visuell auf einer Anzeige darzustellen, sei es zum Beispiel auf einem Display der Vorrichtung selbst und / oder nach einer Datenübertragung auf einer Datenverarbeitungseinrichtung beliebiger Art. Auch können im Zusammenhang mit den erfassten Protokolldaten Auswertealgorithmen Anwendung finden, zum Beispiel zur Auswertung von erfassten Messwerten. Dies kann auf der Vorrichtung selbst und / oder einer anderen Datenverarbeitungseinrichtung erfolgen, zum Beispiel auf einem zentralen Server.

Eine bevorzugte Weiterbildung der Erfindung sieht vor, dass die Programmanweisungen weiterhin konfiguriert sind, beim Abarbeiten durch den Prozessor das Ausgeben des Benutzer-Starthinweises zu bewirken, wenn beim Überwachen des zeitlichen Abstandes zwischen dem aktuellen Datum und dem zukünftigen Ereignis festgestellt wird, dass ein zeitlicher Mindestabstand zwischen dem aktuellen Datum und dem zukünftigen Ereignis erreicht oder unterschritten ist. Ergänzend kann vorgesehen sein, dass dem zeitlichen Mindestabstand in bestimmter Zeit vorgeschaltet ein Vorabhinweis ausgegeben wird. Hierdurch kann der Patient zum Beispiel vorab auf eine später anstehende Messwerterfassung hingewiesen werden.

Bei einer zweckmäßigen Ausgestaltung der Erfindung kann vorgesehen sein, dass der zeitliche Mindestabstand in dem ausgewählten medizinischen Protokoll angegeben ist. Bei dieser Ausführungsform geben die unterschiedlichen medizinischen Protokolle alle oder zum Teil selbst bereits einen zeitlichen Mindestabstand an, zu dem das Ausgeben des Benutzer-Starthinweises spezifisch für ein bestimmtes Protokoll zu erfolgen hat.

Eine vorteilhafte Ausführungsform der Erfindung sieht vor, dass die Programmanweisungen weiterhin konfiguriert sind, beim Abarbeiten durch den Prozessor das Bestimmen des zeitlichen Mindestabstandes ausgehend von dem in dem ausgewählten medizinischen Protokoll angegebenen Protokollzeitraum zu bewirken. Bei dieser Ausführungsform ist in einer Ausgestaltung vorgesehen, dass beim Ausführen der Anweisungen des Applikationsmoduls die elektronische Information über den Protokollzeitraum in dem medizinischen Protokoll ausgewertet wird. Hiervon ausgehend wird dann der zeitliche Mindestabstand bestimmt, der mindestens gleich dem Protokollzeitraum sein muss. Für die Ausgabe des Benutzer-Starthinweises kann aber auch ein zeitlicher Vorlauf bestimmt werden, indem zum Protokollzeitraum ein Zeitraum addiert wird, beispielsweise Stunden oder Tage oder ein Wochenende. Der Protokollzeitraum kann zum Beispiel mehrere Tage umfassen, zum Beispiel drei bis fünf Tage, weshalb dann die Protokolleintragungen zweckmäßigerweise innerhalb von drei bis fünf Tagen vor dem zukünftigen, Protokoll auslösenden Ereignis, zum Beispiel einem Arztbesuch, auszuführen sind.

Bevorzugt sieht eine Fortbildung der Erfindung vor, dass die Programmanweisungen weiterhin konfiguriert sind, beim Abarbeiten durch den Prozessor das Bestimmen des zeitlichen Mindestabstandes unter Berücksichtigung benutzerspezifischer Informationen zu bewirken. So kann vorgesehen sein, bei der Bestimmung des zeitlichen Mindestabstandes, welcher unter Berücksichtigung des im medizinischen Protokoll angegebenen Protokollzeitraumes ermittelt wird, benutzerspezifische Angaben, die wahlweise zuvor vom Benutzer angefordert wurden oder bereits in elektronischer Form vorliegen, einzubeziehen. So kann vorgesehen sein, bei der Bestimmung des zeitlichen Mindestabstandes zu dem Protokollzeitraum eine Zeitdauer zu addieren, die vom Alter und / oder Indikatoren für den Krankheitszustand des Patienten abhängt. Auch kann vorgesehen sein, hierbei spezielle Lebensumstände des Benutzers zu berücksichtigen, zum Beispiel eine Tätigkeit in Schichtarbeit.

Bei einer vorteilhaften Ausgestaltung der Erfindung kann vorgesehen sein, dass die Programmanweisungen weiterhin konfiguriert sind, beim Abarbeiten durch den Prozessor nach dem Zeitpunkt für den Benutzer-Starthinweis und vor zukünftigen Ereignis das Ausgeben zumindest eines Erinnerungs-Starthinweises betreffend das Beginnen des Protokollzeitraumes zu bewirken. Zeitpunkte für das Ausgeben des zumindest einen Erinnerungs-Starthinweises können bereits im medizinischen Protokoll angegeben sein. Im Übrigen gelten die vorangehend in Verbindung mit dem zeitlichen Timing des Ausgebens des Benutzer-Starthinweises gemachten Erläuterungen zu Ausgestaltungsvarianten entsprechend für das Ausgeben des zumindest einen Erinnerungs-Starthinweises.

Eine Weiterbildung der Erfindung kann vorsehen, dass der Benutzer-Starthinweis und / oder der zumindest eine Erinnerungs-Starthinweis eine Information betreffend einen Startzeitpunkt für das Protokollregime nach dem ausgewählten medizinischen Protokoll umfassen. Bei dieser Ausgestaltung ist zum Beispiel vorgesehen, dass der Benutzer-Starthinweis und / oder der zumindest eine Erinnerungs-Starthinweis den Startzeitpunkt mit Tag und Zeit angeben.

Bevorzugt sieht eine Fortbildung der Erfindung vor, dass die Programmanweisungen weiterhin konfiguriert sind, beim Abarbeiten durch den Prozessor für die Protokollereignisse jeweils das Ausgeben eines Benutzer-Ereignishinweises, welcher einem der Protokollereignisse zugeordnet ist, betreffend das Anstehen des Protokollereignisses zu bewirken. Für den Fall, dass es sich bei dem Protokollereignis um eine Messwerterfassung handelt, umfasst der Benutzer-Ereignishinweis, bei dem es sich dann um einen Benutzer-Messhinweis handelt, zum Beispiel einen Hinweis zu Messbedingungen. So kann der Benutzer beispielsweise darauf hingewiesen werden, eine bestimmte Zeit vor dem Messung keine Nahrung aufzunehmen. Der Zeitpunkt oder der zeitliche Abstand für das Ausgeben des Benutzer-Ereignishinweises, welcher einem der gemäß Protokoll abzuarbeitenden Ereignisse, zum Beispiel Messwerterfassungen, zugeordnet ist, kann bereits im ausgewählten medizinischen Protokoll festgelegt sein. Im Übrigen gelten die vorangehend im Zusammenhang mit Ausgestaltungen zu dem Benutzer-Starthinweis gemachten Ausführungen entsprechend.

Eine bevorzugte Weiterbildung der Erfindung sieht vor, dass sich der Benutzer-Ereignishinweis gemäß zugeordneten Ereignisinformationen in dem medizinischen Protokoll auf ein dem Protokollereignis zeitlich vor- oder nachgelagertes, nutzerspezifisches Ereignis bezieht. Nutzerspezifische Ereignisse können zum Beispiel eine Mahlzeit und / oder eine sportliche Betätigung des Patienten sein. Der Benutzer-Ereignishinweis gibt dem Patienten dann protokollspezifische Hinweise darüber, wie das betreffende Protokollereignis, also zum Beispiel eine Messwerterfassung, in zeitlicher Relation zu dem nutzerspezifischen Ereignis steht. So kann zum Beispiel ein fester zeitlicher Abstand angegeben werden, mit dem eine zu protokollierende Messung vor und / oder nach dem nutzerspezifischen Ereignis ausgeführt werden soll, zum Beispiel einem zeitlichen Abstand zu einer Nahrungsaufnahme.

Bei einer zweckmäßigen Ausgestaltung der Erfindung kann vorgesehen sein, dass die Programmanweisungen weiterhin konfiguriert sind, beim Abarbeiten durch den Prozessor nach dem Zeitpunkt des Ausgebens des Benutzer-Ereignishinweises und vor einem dem Protokollereignis zugeordneten Ereigniszeitpunkt für die Protokollereignisse jeweils das Ausgeben zumindest eines Erinnerungs-Ereignishinweises, welcher dem Protokollereignis zugeordnet ist, betreffend das Anstehen des Protokollereignisses zu bewirken. Dieses bedeutet zum Beispiel, dass der Benutzer zunächst auf ein Protokollereignis hingewiesen wird (Benutzer-Ereignishinweis), zum Beispiel eine anstehende Messung. Bevor die Messung dann tatsächlich ansteht, wird der Benutzer hieran zumindest einmal erinnert (Erinnerungs-Ereignishinweis). Das Ausgeben des zumindest einen Erinnerungs-Ereignishinweises definierende Zeitangaben können bereits vom medizinischen Protokoll umfasst sein. Auch hier gelten im Übrigen die in Verbindung mit dem Benutzer-Starthinweis und / oder dem zumindest einen Erinnerungs-Starthinweis hinsichtlich unterschiedlicher Ausführungsformen gemachten Erläuterungen entsprechend.

Eine vorteilhafte Ausführungsform der Erfindung sieht vor, dass der Benutzer-Ereignishinweis und / oder der zumindest eine Erinnerungs-Ereignishinweis eine Information betreffend den Ereigniszeitpunkt umfassen. Es gelten hier die oben in Verbindung mit dem Benutzer-Starthinweis und / oder dem zumindest einen Erinnerungs-Starthinweis gemachten Erläuterungen entsprechend.

Bevorzugt sieht eine Fortbildung der Erfindung vor, dass die Programmanweisungen weiterhin konfiguriert sind, beim Abarbeiten durch den Prozessor ein Erfassen und ein elektronisches Speichern von den Protokollereignissen zugeordneten Protokollinformationen zu bewirken. Bei dieser Ausführungsform kann vorgesehen sein, über die Benutzerschnittstelle und / oder die Anzeigeeinrichtung eine Protokollerfassungsmaske, zum Beispiel eine Messwerterfassungsmaske, dem Patienten zur Verfügung zu stellen, insbesondere zur strukturierten Erfassung von Blutzuckermesswerten. Hierbei kann wahlweise vorgesehen sein, ergänzende nutzerspezifische Informationen zu erfassen, zum Beispiel betreffend Mahlzeiten und / oder sportliche Betätigungen, für die ein zeitlicher Bezug zu den Messwerten bestehen kann.

Eine zweckmäßige Weiterbildung der Erfindung sieh vor, dass die medizinischen Protokolle als strukturierten Messwerterfassungsprotokoll gebildet sind, welche jeweils eine Gruppe von zusammenhängenden Messereignissen angeben, die über einen Messzeitraum einem Messregime entsprechend zeitlich beabstandet sind, und dass der Benutzer-Starthinweis das Beginnen einer Messwerterfassung dem strukturierten Messwerterfassungsprotokoll entsprechend zu einem Zeitpunkt betrifft, welcher in zeitlicher Hinsicht wenigstens um den Messzeitraum vor dem zukünftigen Ereignis liegt.

Bei einer vorteilhaften Ausgestaltung der Erfindung kann vorgesehen sein, dass der Prozessor in einem Gerät ausgewählt aus der folgenden Gerät gebildet ist: Datenverarbeitungseinrichtung wie Laptop, Datenkommunikationsgerät wie Mobilfunktelefon und medizinisches Mess- oder Analysegerät wie Blutzuckermessgerät. Die Vorrichtung oder das System zum Unterstützten eines Patienten mit einer chronischen / nicht-chronischen Krankheit kann verschiedene Ausprägungen annehmen. Das Applikationsmodul mit den Programmanweisungen kann mittels geeigneter Software in unterschiedlichen Gerätetypen oder -systemen implementiert werden. Hierzu gehören beispielsweise auch Applikationen auf Mobilfunktelefonen. Aber auch medizinische Mess- oder Analysegeräte selbst können dem beschriebenen Management der Protokolldatenerfassung in Bezug auf einen zukünftigen Arzttermin realisieren.

In Verbindung mit dem Verfahren zum Betreiben einer Vorrichtung zur Unterstützung eines Patienten mit einer chronischen oder einer nicht-chronischen Krankheit, insbesondere Diabetes, gelten die vorangehend im Zusammenhang mit der Vorrichtung zum Unterstützen des Patienten gemachten Erläuterungen zu vorteilhaften Ausgestaltungen entsprechend.

### Beschreibung bevorzugter Ausführungsbeispiele der Erfindung

Die Erfindung wird im Folgenden anhand von bevorzugten Ausführungsbeispielen unter Bezugsnahme auf Figuren einer Zeichnung näher erläutert. Hierbei zeigen:
- Fig. 1: eine schematische Darstellung einer Anordnung mit einer Vorrichtung zum Unterstützen eines Patienten einer chronischen oder einer nicht-chronischen Krankheit, insbesondere Diabetes,
- Fig. 2: eine schematische Blockdarstellung zu Prozessschritten beim Verarbeiten von Programmanweisungen in einem Prozessor und
- Fig. 3: eine schematische Zeitstrahldarstellung zu Prozessschritten beim Verarbeiten von Programmanweisungen in einem Prozessor

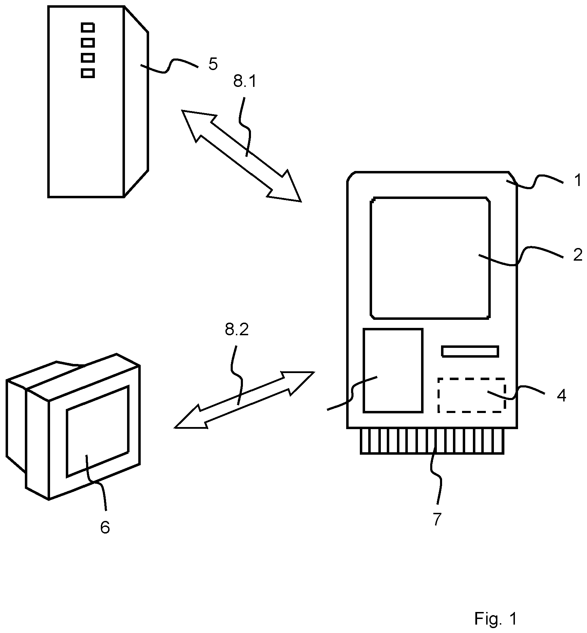

Die Fig. 1 zeigt eine schematische Darstellung einer Anordnung mit einer Vorrichtung 1 zum Unterstützen eines Patienten einer chronischen oder einer nicht-chronischen Krankheit, insbesondere Diabetes. Die Vorrichtung 1 verfügt über eine Anzeigeeinrichtung 2 in Form eines Displays, eine Benutzerschnittstelle, die in der dargestellten Ausführungsform eine Tastatur 3 aufweist, sowie einen Prozessor 4, welcher mit der Anzeigeeinrichtung 2 sowie der Tastatur 3 datentechnisch verbunden ist, so dass zwischen den Elementen elektronische Daten austauschbar sind. Bei der Anordnung nach Fig. 1 sind weiterhin eine Servereinrichtung 5 sowie eine weitere Anzeigeeinrichtung 6 gezeigt, die funktionell an die Vorrichtung 1 koppeln, derart, dass elektronische Daten über eine zugeordnete Schnittstelle 7 der Vorrichtung 1 sowie eine kabellose und / oder eine kabelgebundene Kommunikationsverbindung 8.1, 8.2 austauschbar sind.

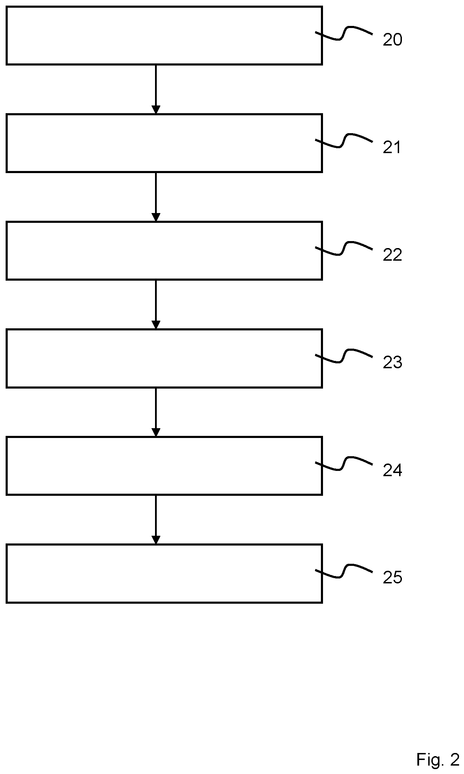

Die Vorrichtung 1 ist mittels Hard- und Software konfiguriert, einen Patienten mit einer chronischen oder einer nicht-chronischen Krankheit in der folgenden Art und Weise zu unterstützen. Hierzu wird für eine Ausführungsform Bezug genommen auf eine schematische Darstellung in Fig. 2.

Die Vorrichtung 1 kann unterschiedliche Gerätearten implementieren, beispielsweise einen mobilen Computer, ein Mobilfunktelefon oder ein Blutzuckermessgerät. Mithilfe eines softwaremäßigen Applikationsmoduls, welches Programmanweisungen umfasst, ist die Vorrichtung 1 konfiguriert, beim Ausführen der Programmanweisungen mit dem Prozessor 4 Folgendes zu bewirken. In einem Schritt 20 wird Zeitinformation betreffend eine zukünftige medizinische Konzentration eines Patienten erfasst, also zum Beispiel Zeitinformation für einen zukünftigen Arzttermin. Das Erfassen der Zeitinformation kann beispielsweise dadurch erfolgen, dass der Benutzer oder Patient die Zeitdaten mittels der Tastatur 3 eingibt.

Im Folgenden Schritt 21 wird mit Hilfe des Prozessors 4 ein strukturiertes Messwertprotokoll ausgewählt. Diese Auswahl kann aus einer Menge strukturierter Messwerterfassungsprotokolle vorgenommen werden, die in der Vorrichtung 1 selbst gespeichert sind. Aber auch ein Zugriff auf eine Menge von strukturierten Messwerterfassungsprotokollen in der Servereinrichtung 5 kann hierbei stattfinden. Das strukturierte Messwerterfassungsprotokoll gibt insbesondere eine Menge von zusammenhängenden Messereignissen an, die über einen Messzeitraum einen protokollspezifischen Regime entsprechend zeitlich beabstandet sind. Der Messzeitraum kann sich über einen oder mehrere Tage erstrecken. Grundsätzlich sind beliebige Messzeiträume möglich. Das strukturierte Messwerterfassungsprotokoll gibt an, wie viele Messereignisse in dem angegebenen Messzeitraum stattfinden sollen. Des Weiteren wird der Zeitpunkt für die Messereignisse durch das strukturierte Messwerterfassungsprotokoll vorgeben. Dieses kann durch konkrete Datums- und Zeitangaben erfolgen. Auch kann vorgesehen sein die Messereignisse, d. h. das Erfassen von Messwerten durch den Patienten, in Beziehung zur nutzerspezifischen Ereignissen zu setzen, beispielsweise eine Mahlzeit oder eine sportliche Aktivität des Benutzers.

Sodann wird in einem Schritt 22 von der Vorrichtung 1 der zeitliche Abstand zwischen einem fortlaufenden aktuellen Datum und dem Datum für den zukünftigen Arztbesuch überwacht.

Wird hierbei festgestellt, dass der zeitliche Abstand zwischen dem aktuellen Datum und dem zukünftigen Arzttermin erreicht oder unterschritten ist, so wird in einem Schritt 23 ein Benutzer-Starthinweis von dem Prozessor erzeugt und ausgegeben. Der Benutzer-Starthinweis gibt dem Patienten Informationen über den Zeitpunkt des Beginns der Messwerterfassung dem strukturierten Messwerterfassungsprotokoll entsprechend. D. h. der Benutzer wird darüber informiert, wann der dem zukünftigen Arzttermin zugeordnete Messwertprozess gemäß dem strukturierten Messwerterfassungsprotokoll zu beginnen hat. Die Ausgabe des Benutzer-Starthinweises kann über die Anzeigeeinrichtung 2 und / oder die weitere Anzeigeeinrichtung 6 erfolgen. Dieses bedeutet, dass die Ausgabe des Benutzer-Starthinweises durch die Vorrichtung 1 selbst und / oder ein hiervon getrenntes Gerät erfolgen kann.

In einem Schritt 24 ist optional vorgesehen, den Benutzer der Vorrichtung 1 zwischen dem Zeitpunkt der Ausgabe des Benutzer-Starthinweises und dem tatsächlichen Anfangszeitpunkt der Messwerterfassung ein oder mehrere Erinnerungs-Starthinweise zu geben, um den Patienten an die anstehende Messwerterfassung gemäß dem strukturierten Messwerterfassungsprotokoll zu erinnern.

In einem Schritt 25 gemäß Fig. 2 wird schließlich ein Benutzer-Messhinweis erzeugt und ausgegeben, mit dem der Benutzer der Vorrichtung 1 nun auf eine konkrete Messung gemäß dem strukturierten Messwerterfassungsprotokoll hingewiesen wird.

Das sammeln der Messwertdaten kann dann auf verschiedene Art und Weise erfolgen. So kann vorgesehen sein, dem Patienten in Papierform ein Messprotokoll zu Verfügung zu stellen, in welches er die dem strukturierten Messwerterfassungsprotokoll gemessenen Messwerte, zum Beispiel in Form von Blutzuckermesswerten, handschriftlich notiert. Es kann alternativ oder ergänzend vorgesehen sein, dass Mittels der Vorrichtung 1 und / oder einer hiervon getrennten Datenverarbeitungsanlage eine elektronische Messprotokollmaske bereitgestellt wird, in die der Benutzer dann die gemessenen Werte einträgt. Unabhängig von der konkreten Art des Sammeln und Speicherns der Messwerte steht damit dann Information für den Arzttermin zur Verfügung, so dass das medizinische Personal diese Informationen auswerten kann. Es wird so sichergestellt, dass die Messwerterfassung in gewünschter Art und Weise erfolgt, insbesondere hinsichtlich der Häufigkeit und des zeitlichen Abstandes zwischen den Messereignissen.

Die Erfindung wurde vorangehend unter Bezugnahme auf Ausführungsbeispiele im Zusammenhang insbesondere mit einem strukturierten Messwerterfassungsprotokoll beschrieben. Die Aspekte der Erfindung können aber auch mit anderen medizinischen Protokollen eingesetzt werden, die diagnostischen und / oder therapeutischen Zwecken dienen. So können alternativ oder ergänzend andere Protokollereignisse einem medizinischen Protokoll entsprechend erfasst werden, zum Beispiel eine Medikamenteneinnahme, eine Nahrungsaufnahme und / oder Symptombeobachtungen des Patienten.

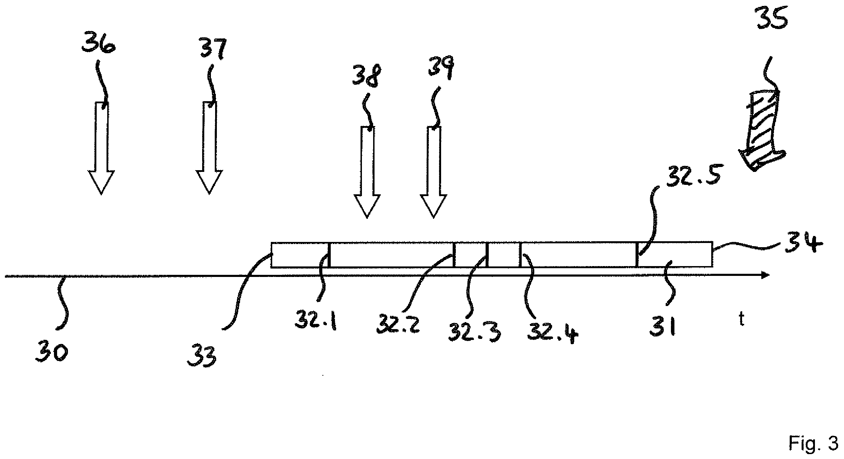

Fig. 3 zeigt eine schematische Darstellung des zeitlichen Ablaufs von Prozessschritten. Längs eines Zeitstrahls 30 sind ein Protokollzeitraum 31 sowie hierin liegende Protokollereignisse 32.1, ..., 32.5 dargestellt. Der Protokollzeitraum 31 erstreckt sich von einem Beginn des Protokolls 33 bis zum Ende des Protokolls 34. Auslösend für das medizinische Protokoll, also zum Beispiel ein Messwerterfassungsprotokoll, bei dem die Protokollereignisse 32.1, ..., 32.5 einer jeweiligen Messwerterfassung entsprechen, ist ein zukünftiges Ereignis 35, bei dem es sich beispielsweise um einen medizinischen Konsultationstermin bei einem Arzt handelt. Vor dem Beginn des Protokolls 33 wird ein Benutzer-Starthinweis 36 ausgegeben. Der Benutzer wird auf diese Weise über das anstehende medizinische Protokoll informiert. Gemäß der schematischen Darstellung in Fig. 3 ist eine Erinnerung des Benutzers an das anstehende medizinische Protokoll mit einem Erinnerungs-Starthinweis 37 vorgesehen.

Während des Protokollzeitraums ist dann bei der Ausgestaltung in Fig. 3 noch vorgesehen, den Benutzer auf ein bestimmtes Protokollereignis 32.2 mit einem Benutzer-Ereignishinweis 38 hinzuweisen. Beispielsweise wird der Benutzer auf diese Weise über eine anstehende Blutzuckermessung informiert. Dieses kann beispielsweise in visueller und / oder akustischer Form der Signalisierung erfolgen. Bei dem dargestellten Ausführungsbeispiel in Fig. 3 ist sodann weiterhin ein Erinnerungs-Ereignishinweis 39 vorgesehen, mit dem der Benutzer an das anstehende Protokollereignis 32.2 erinnert wird.

Wie sich aus Fig. 3 ergibt, können die Protokollereignisse 32.1, ..., 32.5 unterschiedliche oder gleiche zeitliche Abstände voneinander aufweisen.

## Patentansprüche

1. Vorrichtung zum Unterstützen eines Patienten mit einer chronischen oder einer nicht-chronischen Krankheit, insbesondere Diabetes, mit:
- einer Anzeigeeinrichtung (2),
- einer Benutzerschnittstelle (3),
- einem Prozessor (4), der an die Anzeigeeinrichtung (2) und die Benutzerschnittstelle (3) datentechnisch koppelt, und
- einem mittels Software implementierten Applikationsmodul mit Programmanweisungen, die konfiguriert sind, beim Abarbeiten durch den Prozessor (4) Folgendes zu bewirken:
- Bereitstellen von medizinischen Protokollen für diagnostische und / oder therapeutische Zwecke
- Auswählen eines der medizinischen Protokolle, welches eine Gruppe von zusammenhängenden Protokollereignissen angibt, die über einen Protokollzeitraum einem Protokollregime entsprechend zeitlich beabstandet sind, wobei sich der Protokollzeitraum von einem Beginn des medizinischen Protokolls bis zu einem Ende des medizinischen Protokolls erstreckt, und
- Erfassen eines Termins betreffend ein zukünftiges Ereignis, wobei das zukünftige Ereignis Auslöser für das ausgewählte medizinische Protokoll ist,
- Überwachen eines zeitlichen Abstandes zwischen einem aktuellen Datum und dem Termin des zukünftigen Ereignisses und
- Ausgeben eines Benutzer-Starthinweises, wobei der Benutzer-Starthinweis ein akustisches, visuelles und / oder taktiles Signal ist,
**dadurch gekennzeichnet, dass**
- in dem ausgewählten medizinischen Protokoll ein zeitlicher Mindestabstand angegeben ist, der wenigstens dem Protokollzeitraum entspricht, und
- das zukünftige Ereignis einen Zeitpunkt bestimmt, zu dem das ausgewählte medizinische Protokoll beendet sein soll,
wobei das Applikationsmodul weiterhin Programmanweisungen umfasst, die konfiguriert sind, beim Abarbeiten durch den Prozessor (4) Folgendes zu bewirken:
- Bestimmen des zeitlichen Mindestabstands ausgehend von dem in dem ausgewählten medizinischen Protokoll angegebenen Protokollzeitraum und unter Berücksichtigung benutzerspezifischer Informationen, die zuvor vom Patienten angefordert wurden oder bereits in elektronischer Form vorliegen, wobei hierbei zum Protokollzeitraum eine Zeitdauer addiert wird, und
- Ausgeben des Benutzer-Starthinweises, welcher das Beginnen des medizinischen Protokolls betrifft, wenigstens um den zeitlichen Mindestabstand vor dem Termin des zukünftigen Ereignisses.

2. Vorrichtung nach Anspruch 1, **dadurch gekennzeichnet, dass** die Programmanweisungen weiterhin konfiguriert sind, beim Abarbeiten durch den Prozessor (4) nach dem Zeitpunkt für den Benutzer-Starthinweis und vor dem zukünftigen Ereignis das Ausgeben zumindest eines Erinnerungs-Starthinweises betreffend das Beginnen des Protokollzeitraumes zu bewirken.

3. Vorrichtung nach Anspruch 1 oder 2 mindestens einem der vorangehenden Ansprüche, **dadurch gekennzeichnet, dass** der Benutzer-Starthinweis und / oder der zumindest eine Erinnerungs-Starthinweis eine Information betreffend einen Startzeitpunkt für das Protokollregime nach dem ausgewählten medizinischen Protokoll umfassen.

4. Vorrichtung nach mindestens einem der vorangehenden Ansprüche, **dadurch gekennzeichnet, dass** die Programmanweisungen weiterhin konfiguriert sind, beim Abarbeiten durch den Prozessor (4) für die Protokollereignisse jeweils das Ausgeben eines Benutzer-Ereignishinweises, welcher einem der Protokollereignisse zugeordnet ist, betreffend das Anstehen des Protokollereignisses zu bewirken.

5. Vorrichtung nach Anspruch 4, **dadurch gekennzeichnet, dass** sich der Benutzer-Ereignishinweis gemäß zugeordneten Ereignisinformationen in dem medizinischen Protokoll auf ein dem Protokollereignis zeitlich vor- oder nachgelagertes, nutzerspezifisches Ereignis bezieht.

6. Vorrichtung nach Anspruch 4 oder 5, **dadurch gekennzeichnet, dass** die Programmanweisungen weiterhin konfiguriert sind, beim Abarbeiten durch den Prozessor (4) nach dem Zeitpunkt des Ausgebens des Benutzer-Ereignishinweises und vor einem dem Protokollereignis zugeordneten Ereigniszeitpunkt für die Protokollereignisse jeweils das Ausgeben zumindest eines Erinnerungs-Ereignishinweises, welcher dem Protokollereignis zugeordnet ist, betreffend das Anstehen des Protokollereignisses zu bewirken.

7. Vorrichtung nach mindestens einem der Ansprüche 4 bis 6, dadurch **gekenn - zeichnet,** dass der Benutzer-Ereignishinweis und / oder der zumindest eine Erinnerungshinweis eine Information betreffend den Ereigniszeitpunkt umfassen.

8. Vorrichtung nach mindestens einem der vorangehenden Ansprüche, dadurch **ge - kennzeichnet,** dass die Programmanweisungen weiterhin konfiguriert sind, beim Abarbeiten durch den Prozessor (4) ein Erfassen und ein elektronisches Speichern von den Protokollereignissen zugeordneten Protokollinformationen zu bewirken.

9. Vorrichtung nach mindestens einem der vorangehenden Ansprüche, dadurch **ge - kennzeichnet**, dass die medizinischen Protokolle als strukturierten Messwerterfassungsprotokoll gebildet sind, welche jeweils eine Gruppe von zusammenhängenden Messereignissen angeben, die über einen Messzeitraum einem Messregime entsprechend zeitlich beabstandet sind, und dass der Benutzer-Starthinweis das Beginnen einer Messwerterfassung dem strukturierten Messwerterfassungsprotokoll entsprechend zu einem Zeitpunkt betrifft, welcher in zeitlicher Hinsicht wenigstens um den Messzeitraum vor dem zukünftigen Ereignis liegt.

10. Vorrichtung nach mindestens einem der vorangehenden Ansprüche, **dadurch gekennzeichnet, dass** der Prozessor in einem Gerät ausgewählt aus der folgenden Gruppe gebildet ist: Datenverarbeitungseinrichtung wie Laptop, Datenkommunikationsgerät wie Mobilfunktelefon und medizinisches Mess- oder Analysegerät wie Blutzuckermessgerät.

11. Verfahren zum Betreiben einer Vorrichtung zur Unterstützung eines Patienten mit einer chronischen oder einer nicht-chronischen Krankheit, insbesondere Diabetes, mit einer Anzeigeeinrichtung (2), einer Benutzerschnittstelle (3), einem Prozessor (4), der an die Anzeigeeinrichtung (2) und die Benutzerschnittstelle (3) datentechnisch koppelt, und einem. als Software implementierten Applikationsmodul mit Programmanweisungen, wobei das Verfahren beim Abarbeiten der Programmanweisungen durch den Prozessor (4) die folgenden Schritte umfasst:
- Bereitstellen von medizinischen Protokollen für diagnostische und / oder therapeutische Zwecke
- Auswählen eines der medizinischen Protokolle, welches eine Gruppe von zusammenhängenden Protokollereignissen angibt, die über einen Protokollzeitraum einem Protokollregime entsprechend zeitlich beabstandet sind, wobei sich der Protokollzeitraum von einem Beginn des medizinischen Protokolls bis zu einem Ende des medizinischen Protokolls erstreckt, und
- Erfassen eines Termins betreffend ein zukünftiges Ereignis, wobei das zukünftige Ereignis Auslöser für das ausgewählte medizinische Protokoll ist,
- Überwachen eines zeitlichen Abstandes zwischen einem aktuellen Datum und dem Termin des zukünftigen Ereignisses und
- Ausgeben eines Benutzer-Starthinweises, wobei der Benutzer-Starthinweis ein akustisches, visuelles und / oder taktiles Signal ist,
**dadurch gekennzeichnet, dass**
- in dem ausgewählten medizinischen Protokoll ein zeitlicher Mindestabstand angegeben ist, der wenigstens dem Protokollzeitraum entspricht,
- das zukünftige Ereignis einen Zeitpunkt bestimmt, zu dem das ausgewählte medizinische Protokoll beendet sein soll,
wobei das Verfahren beim Abarbeiten der Programmanweisungen durch den Prozessor (4) die weiteren folgenden Schritte umfasst:
- Bestimmen des zeitlichen Mindestabstands ausgehend von dem in dem ausgewählten medizinischen Protokoll angegebenen Protokollzeitraum und unter Berücksichtigung benutzerspezifischer Informationen, die zuvor vom Patienten angefordert wurden oder bereits in elektronischer Form vorliegen, wobei hierbei zum Protokollzeitraum eine Zeitdauer addiert wird, und
- Ausgeben des Benutzer-Starthinweises, welcher das Beginnen des medizinischen Protokolls betrifft, wenigstens um den zeitlichen Mindestabstand vor dem Termin des zukünftigen Ereignisses.

## Claims

1. Device for supporting a patient with a chronic or non-chronic disease, particularly diabetes, comprising:
- a display device (2);
- a user interface (3);
- a processor (4) that is coupled with the display device (2) and the user interface (3) for data processing purposes; and
- a software-implemented application module with program instructions which are configured to effect the following when executed by the processor (4),
- providing medical protocols for diagnostic and/or therapeutic purposes,
- selecting one of the medical protocols which specifies a group of related protocol events that occur at separate points in time during a protocol period corresponding to a protocol regimen, wherein the protocol period extends from a start of the medical protocol to an end of the medical protocol, and
- recording a date relating to a future event, wherein the future event is the initiator of the selected medical protocol,
- monitoring a chronological interval between a current date and the date of the future event, and
- issuing a user start notice, wherein the user start notice is an acoustic, visual and/or tactile signal,
**characterised in that**
- a minimum time interval is specified in the selected medical protocol which at least corresponds to the protocol period, and
- the future event determines a point in time at which the selected medical protocol is to be terminated,
wherein the application module further comprises program instructions that are configured to effect the following when executed by the processor (4):
- determine the minimum time interval starting from the protocol period specified in the selected medical protocol and taking into account user-specific information which was requested from the patient previously or is already available in electronic form, wherein additional time is added to the protocol period in this eventuality, and
- issue the user start notice relating to the start of the medical protocol at least as long as the minimum time period before the date of the future event.

2. Device according to claim 1, **characterised in that** upon execution by the processor (4) the program instructions are further configured to effect the issue of a start notice reminder relating to the beginning of the protocol period after the time for the user start notice and before the future event.

3. Device according to claim 1 or 2 at least one of the preceding claims, **characterised in that** the user start notice and/or the at least one start notice reminder comprise information relating to a starting time point for the protocol regimen according to the selected medical protocol.

4. Device according to at least one of the preceding claims, **characterised in that** upon execution by the processor (4) the program instructions are further configured to effect the issue of one user event notice assigned to each protocol event relating to the pending status of the protocol event.

5. Device according to claim 4, **characterised in that** the user event notice relates to a user-specific event that is scheduled to take place before or after the protocol event according to assigned event information in the medical protocol.

6. Device according to claim 4 or 5, **characterised in that** upon execution by the processor (4) the program instructions are further configured to effect the issue of at least one event reminder notice assigned to each protocol event and relating to the pending status of the protocol event after the time of issue of the user event notice and before an event time assigned to the protocol event.

7. Device according to at least one of claims 4 to 6, **characterised in that** the user event notice and/or the at least one reminder notice comprise information relating to the event time.

8. Device according to at least one of the preceding claims, **characterised in that** upon execution by the processor (4) the program instructions are further configured to effect the capture and electronic storage of protocol information assigned to the protocol events.

9. Device according to at least one of the preceding claims, **characterised in that** the medical protocols are created as a structured measurement value data entry log, each of which lists a group of related measurement events occurring at separate times over a measurement period corresponding to a measurement regimen, and that the user start notice relates to the start of a measurement collection corresponding to the structured measurement value data entry log at a time which is earlier than the future event by at least the measurement period.

10. Device according to at least one of the preceding claims, **characterised in that** processor is created in a device selected from the following group: data processing device such as a laptop, data communication device such as a mobile phone, and medical measurement or analysis device such as blood sugar level measuring device.

11. Method for operating a device for supporting a patient with a chronic or non-chronic disease, particularly diabetes, with a display device (2), a user interface (3), a processor (4) that is coupled with the display device (2) and the user interface (3) for data processing purposes and a software-implemented application module with program instructions, wherein when the processor (4) executes the program instructions the method comprises the following steps:
- providing medical protocols for diagnostic and/or therapeutic purposes,
- selecting one of the medical protocols listing a group of related protocol events that occur at separate times during a protocol period corresponding to a protocol regimen, wherein the protocol period extends from a start of the medical protocol to an end of the medical protocol, and
- recording a date relating to a future event, wherein the future event initiates the selected medical protocol,
- monitoring a time interval between a current date and the date of the future event, and
- issuing a user start notice, wherein the user start notice is an acoustic, visual and/or tactile signal,
**characterised in that**
- a minimum time interval corresponding at least to the protocol period is specified in the selected medical protocol, and
- the future event determines a point in time at which the selected medical protocol is to be terminated,
wherein when the processor (4) executes the program instructions the method comprises the following further steps:
- determining the minimum time interval starting from the protocol period specified in the selected medical protocol and taking into account user-specific information which was requested from the patient previously or is already available in electronic form, wherein a time interval is added to the protocol period in this eventuality, and
- issuing the user start notice relating to the start of the medical protocol at least as long before the date of the future event as the minimum time period.

## Revendications

1. Dispositif destiné à assister un patient atteint d'une maladie chronique ou non chronique, notamment de diabète, comprenant :
- un système d'affichage (2),
- une interface utilisateur (3),
- un processeur (4), qui est informatiquement connecté au système d'affichage (2) et à l'interface utilisateur (3) et
- un module d'application implémenté par logiciel, avec des instructions de programme qui sont configurées pour provoquer ce qui suit, lors de l'exécution par le processeur (4) :
- la mise à disposition de protocoles médicaux à des fins diagnostiques et/ou thérapeutiques
- la sélection de l'un des protocoles médicaux, lequel indique un groupe d'événements protocolaires cohérents, qui sur une période protocolaire sont espacés dans le temps, en correspondance avec un régime protocolaire, la période protocolaire s'étendant d'un début du protocole médical jusqu'à une fin du protocole médical et
- la détection d'un terme concernant un événement futur, l'événement futur étant un déclencheur pour le protocole médical sélectionné
- la surveillance d'un espace temporel entre une date actuelle et le terme de l'événement futur et
- l'édition d'une recommandation de démarrage à l'utilisateur, la recommandation de démarrage à l'utilisateur étant un signal acoustique, visuel et/ou tactile,
**caractérisé en ce que**
- dans le protocole médical sélectionné, il est indiqué un espace temporel minimal qui correspond au moins à la période protocolaire et
- l'événement futur détermine un moment auquel le protocole médical sélectionné est censé être achevé,
le module d'application comprenant par ailleurs des instructions de programme, qui sont configurées pour provoquer ce qui suit, lors de l'exécution par le processeur (4) :
- la détermination de l'espace temporel minimal, sur la base de la période protocolaire indiquée dans le protocole médical sélectionné et sous considération d'informations spécifiques à l'utilisateur, qui ont précédemment été demandées au patient ou qui sont déjà présentes sous forme électronique, alors qu'à cet effet, une durée est additionnée à la période protocolaire et
- l'édition de la recommandation de démarrage à l'utilisateur qui concerne le début du protocole médical, en avance d'au moins l'espace temporel minimal sur le terme de l'événement futur.

2. Dispositif selon la revendication 1, **caractérisé en ce que** les instructions de programme sont configurées en outre pour l'édition lors de l'exécution par le processeur (4), après le moment pour la recommandation de démarrage à l'utilisateur et avant l'événement futur, d'au moins un rappel de la recommandation de démarrage concernant le début de la période protocolaire.

3. Dispositif selon la revendication 1 ou 2, au moins l'une quelconque des revendications précédentes, **caractérisé en ce que** la recommandation de démarrage à l'utilisateur et/ou l'au moins un rappel de la recommandation de démarrage comprennent une information concernant un moment de démarrage pour le régime protocolaire d'après le protocole médical sélectionné.

4. Dispositif selon au moins l'une quelconque des revendications précédentes, **caractérisé en ce que** les instructions de programme sont configurées en outre pour provoquer, lors de l'exécution par le processeur (4) pour les événements protocolaires chaque fois l'édition d'une indication d'événement à l'utilisateur concernant l'imminence de l'événement protocolaire, laquelle est associée à l'un des événements protocolaires.

5. Dispositif selon la revendication 4, **caractérisé en ce que**, conformément à des informations d'événements associés dans le protocole médical, l'indication d'événement à l'utilisateur se rapporte à un événement spécifique à l'utilisateur, antérieur ou postérieur dans le temps à l'événement protocolaire.

6. Dispositif selon la revendication 4 ou 5, **caractérisé en ce que** les instructions de programme sont configurées en outre pour provoquer, lors de l'exécution par le processeur (4), après le moment de l'édition de l'indication d'événement à l'utilisateur et avant le moment d'un événement qui est associé à l'événement protocolaire pour les événements protocolaires chaque fois l'édition d'au moins une indication de rappel d'événement concernant l'imminence de l'événement protocolaire, laquelle est associée à l'événement protocolaire.

7. Dispositif selon au moins l'une quelconque des revendications 4 à 6, **caractérisé en ce que** l'indication d'événement à l'utilisateur et / ou l'au moins une indication de rappel comprennent une information concernant le moment de l'événement.

8. Dispositif selon au moins l'une quelconque des revendications précédentes, **caractérisé en ce que** les instructions de programme sont configurées en outre pour provoquer, lors de l'exécution par le processeur (4) une détection et une sauvegarde électronique d'informations protocolaires associées à des événements protocolaires.

9. Dispositif selon au moins l'une quelconque des revendications précédentes, **caractérisé en ce que** le protocole médical est conçu en tant que protocole structuré d'enregistrement de valeurs mesurées qui indique respectivement un groupe d'événements de mesure cohérents qui sont espacés dans le temps sur une période de mesure, en correspondance avec un régime de mesure et **en ce que** la recommandation de démarrage à l'utilisateur concerne le début d'un enregistrement de valeurs en correspondance avec le protocole structuré d'enregistrement de valeurs mesurées à un moment, qui au niveau temporel est antérieur d'au moins la période de mesure à l'événement futur.

10. Dispositif selon au moins l'une quelconque des revendications précédentes, **caractérisé en ce que** le processeur est formé d'un appareil sélectionné dans le groupe suivant : système de traitement de données, tel qu'un ordinateur portable, appareil de communication de données, tel qu'un téléphone mobile et appareil de mesures et d'analyses médicales, tel qu'un glucomètre.

11. Procédé destiné à faire fonctionner un dispositif d'assistance d'un patient atteint d'une maladie chronique ou non chronique, notamment de diabète, comprenant un système d'affichage (2), une interface utilisateur (3), un processeur (4) qui est informatiquement connecté au système d'affichage (2) et à l'interface utilisateur (3) et un module d'application implémenté en tant que logiciel avec des instructions de programme, lors de l'exécution des instructions de programme par le processeur (4), le procédé comprenant les étapes suivantes:
- de la mise à disposition de protocoles médicaux à des fins diagnostiques et/ou thérapeutiques
- de la sélection de l'un des protocoles médicaux, lequel indique un groupe d'événements protocolaires cohérents, qui sur une période protocolaire sont espacés dans le temps, en correspondance avec un régime protocolaire, la période protocolaire s'étendant d'un début du protocole médical jusqu'à une fin du protocole médical et
- de la détection d'un terme concernant un événement futur, l'événement futur étant un déclencheur pour le protocole médical sélectionné
- de la surveillance d'un espace temporel entre une date actuelle et le terme de l'événement futur et
- de l'édition d'une recommandation de démarrage à l'utilisateur, la recommandation de démarrage à l'utilisateur étant un signal acoustique, visuel et/ou tactile,
**caractérisé en ce que**
- dans le protocole médical sélectionné, il est indiqué un espace temporel minimal qui correspond au moins à la période protocolaire et
- l'événement futur détermine un moment auquel le protocole médical sélectionné est censé être achevé,
lors de l'exécution des instructions de programme par le processeur (4), le procédé comprenant les étapes supplémentaires suivantes :
- de la détermination de l'espace temporel minimal, sur la base de la période protocolaire indiquée dans le protocole médical sélectionné et sous considération d'informations spécifiques à l'utilisateur, qui ont précédemment été demandées au patient ou qui sont déjà présentes sous forme électronique, alors qu'à cet effet, une durée est additionnée à la période protocolaire et
- de l'édition de la recommandation de démarrage à l'utilisateur qui concerne le début du protocole médical, en avance d'au moins l'espace temporel minimal sur le terme de l'événement futur.
